Europäisches Patentamt

European Patent Office

Office européen des brevets

(19)

(11) EP 1 331 009 A1

(12) **EUROPEAN PATENT APPLICATION**
published in accordance with Art. 158(3) EPC

(43) Date of publication:
30.07.2003 Bulletin 2003/31

(21) Application number: 01974752.6

(22) Date of filing: 10.10.2001

(51) Int Cl.7: **A61K 35/84**, A61K 7/26,
A61P 3/00, A23L 1/28,
A23L 1/29

(86) International application number:
PCT/JP01/08876

(87) International publication number:
WO 02/030440 (18.04.2002 Gazette 2002/16)

(84) Designated Contracting States:
AT BE CH CY DE DK ES FI FR GB GR IE IT LI LU
MC NL PT SE TR
Designated Extension States:
AL LT LV MK RO SI

(30) Priority: 11.10.2000 JP 2000311034
11.10.2000 JP 2000311035

(71) Applicant: Kureha Chemical Industry Co., Ltd.
Tokyo 103-8552 (JP)

(72) Inventor: MATSUNAGA, Kenichi
Tokorozawa-shi, Saitama 359-1142 (JP)

(74) Representative: Minderop, Ralph H., Dr. rer. nat.
Cohausz & Florack,
Patentanwälte,
Kanzlerstrasse 8a
40472 Düsseldorf (DE)

(54) **MEDICINAL COMPOSITIONS FOR PROMOTING RECOVERY FROM STRESS LOADING AND NOVEL MATSUTAKE MUSHROOM STRAIN**

(57) A pharmaceutical composition for promoting a recovery from stress comprising *Tricholoma matsutake*, a hot water extract of *Tricholoma matsutake* or a dried product thereof, or an alkaline solution extract of *Tricholoma matsutake* or a dried product thereof, and a pharmaceutically acceptable carrier; and a novel *Tricholoma matsutake* strain FERM BP-7304 are disclosed.

EP 1 331 009 A1

**EP 1 331 009 A1**

**Description**

TECHNICAL FIELD

The present invention relates to a pharmaceutical composition for promoting a recovery from stress and a novel *Tricholoma matsutake* strain. The pharmaceutical composition of the present invention for promoting a recovery from stress may be administered as a medicament or in various forms, for example, eatable or drinkable products such as functional foods or health foods, or feeds. Further, the pharmaceutical composition of the present invention for promoting a recovery from stress may be administered in the form of an oral hygienic composition, which is temporarily kept in the mouth but then spat out, retaining almost none of the components, for example, a dentifrice, a mouthwash agent, a chewing gum, or a collutorium, or in the form of an inhalation drawn in through the nose.

BACKGROUND ART

It is known that *Tricholoma matsutake* contains many physiologically active substances. For example, Japanese Examined Patent Publication (Kokoku) No. 57-1230 and Japanese Patent No. 2767521 disclose various antitumor substances contained in *Tricholoma matsutake*. The above Japanese Examined Patent Publication (Kokoku) No. 57-1230 discloses that emitanine-5-A, emitanine-5-B, emitanine-5-C, and emitanine-5-D, which are separated and purified from a liquid extract obtained by extracting a broth of *Tricholoma matsutake* mycelia with hot water or a diluted alkaline solution, exhibits an activity of inhibiting a proliferation of sarcoma 180 cells. The above Japanese Patent No. 2767521 discloses that a protein with a molecular weight of 0.2 to 0.21 million (a molecular weight of a subunit = 0.1 to 0.11 million) which is separated and purified from an extract of *Tricholoma matsutake* fruit bodies with water exhibits an antitumor activity.

Further, the present inventor found that a hot water extract of *Tricholoma matsutake* or an alkaline solution extract of *Tricholoma matsutake,* or an adsorption fraction of a hot water extract of *Tricholoma matsutake* or an alkaline solution extract of *Tricholoma matsutake* by an anion exchange resin exhibits an immuno-enhancing activity (Japanese Patent Application No. 2000-374).

It is known that stress not only causes a disorder in an adjustment of social life, such as psychosomatic disorder, but also may be a trigger of an attack and a progress of life-style related diseases or the like. An antianxiety agent (tranquilizer) or herbal medicines are prescribed for changes of mind and body caused by stress, whereas a method for effectively treating changes of an immune function caused by stress has not yet been found. To the present inventor's knowledge, no food having an activity of promoting a spontaneous recovery of an immune activity by a release of stress (i.e., activity of promoting a recovery from stress) is known. Further, to the present inventor's knowledge, it is not known that a substance having the activity of promoting a recovery from stress exists in food.

The present inventor made an intensive search for another physiological activity other than the known antitumor activity or immuno-enhancing activity in *Tricholoma matsutake*. As a result, the present inventor newly found that an active ingredient exhibiting the activity of promoting a recovery from stress is contained in *Tricholoma matsutake*. Further, the present inventor made an intensive search for a physiological activity of a novel *Tricholoma matsutake* strain found by the present inventor, and found a novel *Tricholoma matsutake* strain containing an active ingredient exhibiting an excellent activity of promoting a recovery from stress. The present invention is based on the above findings.

DISCLOSURE OF INVENTION

This present invention relates to a pharmaceutical composition for promoting a recovery from stress, comprising *Tricholoma matsutake* [*Tricholoma matsutake (S. Ito & Imai) Sing.*], a hot water extract of *Tricholoma matsutake* or a dried product thereof, or an alkaline solution extract of. *Tricholoma matsutake* or a dried product thereof, and a pharmaceutically acceptable carrier.

Further, the present invention relates to a functional food for promoting a recovery from stress, comprising *Tricholoma matsutake*, a hot water extract of *Tricholoma matsutake* or a dried product thereof, or an alkaline solution extract of *Tricholoma matsutake* or a dried product thereof, alone or optionally with one or more food components.

Further, the present invention relates to an oral hygienic composition for promoting a recovery from stress, comprising *Tricholoma matsutake*, a hot water extract of *Tricholoma matsutake* or a dried product thereof, or an alkaline solution extract of *Tricholoma matsutake* or a dried product thereof, and a carrier for an oral hygienic composition.

Further, the present invention relates to a method for promoting a recovery from stress, comprising administering to a subject in need thereof *Tricholoma matsutake*, a hot water extract of *Tricholoma matsutake* or a dried product thereof, or an alkaline solution extract of *Tricholoma matsutake* or a dried product thereof, in an amount effective therefor.

Further, the present invention relates to the use of *Tricholoma matsutake*, a hot water extract of *Tricholoma matsutake* or a dried product thereof, or an alkaline solution extract of *Tricholoma matsutake* or a dried product thereof, in the manufacture of a pharmaceutical composition for promoting a recovery from stress, a functional food for promoting a recovery from stress, or an oral hygienic composition for promoting a recovery from stress.

Further, the present invention relates to a *Tricholoma matsutake* strain FERM BP-7304, or a mycelium, a broth, or a fruit body of a *Tricholoma matsutake* strain FERM BP-7304.

Further, the present invention relates to a hot water extract of a *Tricholoma matsutake* strain FERM BP-7304 or a dried product thereof, or an alkaline solution extract of a *Tricholoma matsutake* strain FERM BP-7304 or a dried product thereof.

BRIEF DESCRIPTION OF DRAWINGS

Fig. 1 is a photograph showing the results of electrophoresis of PCR products obtained by a RAPD method using known *Tricholoma matsutake* strains represented by Nos. 1 to 6 in Table 1.

Fig. 2 is a photograph showing the results of electrophoresis of PCR products obtained by a RAPD method using known *Tricholoma matsutake* strains represented by Nos. 7 to 11 in Table 1.

Fig. 3 is a photograph showing the results of electrophoresis of PCR products obtained by a RAPD method using the *Tricholoma matsutake* strain FERM BP-7304 of the present invention and known *Tricholoma matsutake* strains represented by Nos. 12 to 13 in Table 1.

Fig. 4 is a photograph showing the results of electrophoresis of PCR products obtained by a RAPD method using the *Tricholoma matsutake* strain FERM BP-7304 of the present invention.

Fig. 5 is a graph showing an activity of promoting a recovery from stress in the mixture of the dry powder of a hot water extract of *Tricholoma matsutake* strain FERM BP-7304 of the present invention and the dry powder of an alkaline solution extract thereof at a ratio of 3.2 : 5.1.

Fig. 6 is a graph showing an activity of promoting a recovery from stress in each dry powder of the *Tricholoma matsutake* strain FERM BP-7304 of the present invention and *Tricholoma matsutake* strains.

Fig. 7 is a graph showing an activity of promoting a recovery from stress in each dry powder of other *Tricholoma matsutake* strains.

Fig. 8 is a graph showing an activity of promoting a recovery from stress in each dry powder of still other *Tricholoma matsutake* strains.

Fig. 9 is a graph showing an activity of promoting a recovery from stress in each dry powder of still other *Tricholoma matsutake* strains.

BEST MODE FOR CARRYING OUT THE INVENTION

The present invention will be explained in detail hereinafter.

The pharmaceutical composition of the present invention for promoting a recovery from stress contains as an active ingredient at least one of

(1) *Tricholoma matsutake* (for example, a mycelium, a broth, or a fruit body of *Tricholoma matsutake*);

(2) a hot water extract of *Tricholoma matsutake* (for example, a hot water extract of the mycelium, the broth, or the fruit body of *Tricholoma matsutake*) or a dried product thereof; or

(3) an alkaline solution extract of *Tricholoma matsutake* (for example, an alkaline solution extract of the mycelium, the broth, or the fruit body of *Tricholoma matsutake*) or a dried product thereof;

and a pharmaceutically or veterinarily acceptable ordinary carrier or diluent.

As the *Tricholoma matsutake* used for preparing the *Tricholoma matsutake,* the hot water extract of *Tricholoma matsutake* or the dried product thereof, or the alkaline solution extract of *Tricholoma matsutake* or the dried product thereof as the active ingredient of the present invention, there may be mentioned, for example, a fruit body or a mycelium of a naturally occurring *Tricholoma matsutake,* or a mycelium (i.e., a cultured mycelium), a broth, or a fruit body obtainable by culturing *Tricholoma* matsutake. The novel *Tricholoma matsutake* strain FERM BP-7304 [*Tricholoma* matsutake *(S.Ito & Imai) Sing.* CM6271] of the present invention is preferably used, because the strain contains the active ingredient exhibiting an excellent activity of promoting a recovery from stress.

As the mycelium of *Tricholoma matsutake* which may be used as the active ingredient in the pharmaceutical composition of the present invention for promoting a recovery from stress, when the mycelia obtainable by cultivation are used, for example, wet mycelia obtained by removing medium from a mixture of the mycelia obtainable by cultivation (i.e., the cultured mycelia) and the medium by an appropriate removing method (such as filtration), dried mycelia obtained by further removing water from the wet mycelia by an appropriate method (such as lyophilization), or powdery dried mycelia obtained by further crushing the dried mycelia may be used. When the naturally occurring mycelia are used, for example, the naturally occurring mycelia without any treatment, dried mycelia obtained by removing water from the naturally occurring mycelia by an appropriate method (such as lyophilization), or powdery dried mycelia obtained by further crushing the dried mycelia may be used.

As the broth of *Tricholoma matsutake* which may be used as the active ingredient in the pharmaceutical composition of the present invention for promoting a recovery from stress, for example, a mixture of the mycelia obtainable by cultivation (i.e., the cultured mycelia) and a medium, a dried broth obtained by removing water from the mixture by an appropriate method (such as lyophilization), or a powdery dried broth obtained by further crushing the dried broth may be used.

As the fruit body of *Tricholoma matsutake* which may be used as the active ingredient in the pharmaceutical composition of the present invention for promoting a recovery from stress, for example, fruit bodies obtainable by cultivation or naturally occurring fruit bodies without any treatment, crushed fruit bodies obtained by crushing the fruit bodies, dried fruit bodies obtained by removing water from the fruit bodies by an appropriate method (such as lyophilization), or powdery dried fruit bodies obtained by further crushing the dried bodies may be used.

The hot water extract of *Tricholoma matsutake* may be prepared, for example, by extracting fruit bodies or mycelia of a naturally occurring *Tricholoma matsutake,* or mycelia (i.e., cultured mycelia), a broth, or fruit bodies obtainable by culturing *Tricholoma matsutake* with hot water.

A temperature of hot water used in the hot water extracting step is not limited, so long as the active ingredient exhibiting an activity of promoting a recovery from stress is sufficiently extracted from *Tricholoma matsutake* in the hot water extract, but is preferably 60 to 100 °C, more preferably 80 to 98 °C.

When mycelia or fruit bodies are used in the hot water extracting step, it is preferable to crush, grind, or pulverize them, to enhance the extraction efficiency.

It is preferable to carry out the hot water extracting step with stirring or shaking, so that an extraction efficiency is enhanced. An extracting time may vary with the form of *Tricholoma matsutake*, for example, a phase of fruit bodies, mycelia, or a broth, a treated form, such as a crushed, ground, or pulverized form, a temperature of hot water, or a treating condition with or without stirring or shrinking, but is for example, 1 to 6 hours, preferably 2 to 3 hours.

The resulting hot water extract liquor may be used as it is, namely, in the state containing insolubles, as the active ingredient of the pharmaceutical composition of the present invention for promoting a recovery from stress. Alternatively, it may be used after removing the insolubles, or after removing the insolubles and then low molecular weight fractions from the extract liquor, as the active ingredient of the pharmaceutical composition of the present invention for promoting a recovery from stress. For example, the insolubles may be removed by centrifuging the hot water extract containing such insolubles, and the resulting supernatant may be used as the active ingredient of the pharmaceutical composition of the present invention for promoting a recovery from stress. Alternatively, after removing the insolubles by centrifuging the hot water extract containing such insolubles, and dialyzing the resulting supernatant to remove low molecular weight fractions, preferably fractions of low molecular weight substances having a molecular weight of 3500 or less, the resulting liquor may be used as the active ingredient of the pharmaceutical composition of the present invention for promoting a recovery from stress. Further, after removing water from the hot water extract liquor (for example, the hot water extract liquor containing insolubles, the supernatant obtainable by centrifuging the hot water extract liquor, or the liquor obtainable by dialyzing the supernatant) by an appropriate method (such as lyophilization), the resulting dried product may be used as the active ingredient of the pharmaceutical composition of the present invention for promoting a recovery from stress.

The alkaline solution extract of *Tricholoma matsutake* or the dried product thereof, as the active ingredient of the pharmaceutical composition of the present invention for promoting a recovery from stress, may be prepared by, for example, a method similar to that for preparing the hot water extract of *Tricholoma matsutake* as mentioned above. More particularly, the procedures of the above-mentioned method for preparing the hot water extract of *Tricholoma matsutake* can be repeated except that an alkaline solution is used instead of hot water, to prepare the alkaline solution extract of *Tricholoma matsutake*. For example, it may be obtained by extracting fruit bodies or mycelia of a naturally occurring *Tricholoma matsutake*, or mycelia (i.e., cultured mycelia), a broth, or fruit bodies obtainable by culturing *Tricholoma matsutake* with an alkaline solution.

The alkaline solution used in the alkaline solution-extracting step may be, for example, but is by no means limited to, an aqueous solution of a hydroxide of an alkaline metal, such as sodium or potassium, particularly sodium hydroxide. A pH value of the alkaline solution is preferably 8 to 13, more preferably 9 to 12. The alkaline solution-extracting step is carried out preferably at 0 to 30 °C, more preferably at 0 to 25 °C. The resulting alkaline solution extract may be used as the active ingredient of the pharmaceutical composition of the present invention for promoting a recovery from stress, without any treatment such as neutralization, or after neutralization.

The pharmaceutical composition of the present invention for promoting a recovery from stress can be administered to an animal, preferably a mammal, more preferably a human, in the form of a mixture of *Tricholoma matsutake* (for example, a mycelium, a broth, or a fruit body of *Tricholoma matsutake*), the hot water extract of *Tricholoma matsutake* (for example, a hot water extract of a mycelium, a broth, or a fruit body of *Tricholoma matsutake*) or the dried product thereof, or the alkaline solution extract of *Tricholoma matsutake* (for example, an alkaline solution extract of a mycelium, a broth, or a fruit body of *Tricholoma matsutake*) or the dried product thereof, with a pharmaceutically or veterinarily acceptable ordinary carrier or diluent.

The active ingredient of the pharmaceutical composition of the present invention for promoting a recovery from stress, that is, *Tricholoma matsutake*, the hot water extract of *Tricholoma matsutake* or the dried product thereof, or the alkaline solution extract of *Tricholoma matsutake* or the dried product thereof, exhibits an activity of promoting a recovery from stress.

The active ingredient of the present invention, that is, *Tricholoma matsutake*, the hot water extract of *Tricholoma matsutake* or the dried product thereof, or the alkaline solution extract of *Tricholoma matsutake* or the dried product thereof, can be administered to a subject in need of promoting a recovery from stress, with or without, but preferably with, a pharmaceutically or veterinarily acceptable ordinary carrier or diluent, in an amount effective therefor.

Further, the active ingredient of the present invention, that is, *Tricholoma matsutake*, the hot water extract of *Tricholoma matsutake* or the dried product thereof, or the alkaline solution extract of *Tricholoma matsutake* or the dried product thereof, can be used in the manufacture of a pharmaceutical composition for promoting a recovery from stress, a functional food for promoting a recovery from stress, or an oral hygienic composition for promoting a recovery from stress.

Generally, when stress is loaded to an animal once or during a certain period, an immune activity in the animal is decreased, but the immune activity is spontaneously recovered after a release of the stress. The "activity of promoting a recovery from stress" as used herein means an activity which promotes the recovery of an immune activity during a period of an immune activity convalescence after a release of stress, in comparison with the spontaneous recovery.

The pharmaceutical composition of the present invention for promoting a recovery from stress can be administered at any time, so long as it can promote the recovery of an immune activity briefly lowered by stress. It can be administered, for example, before a loading of stress, during a loading of stress, and/or during a period of an immune activity convalescence after a release of stress.

In this connection, the "activity of promoting a recovery from stress" in the present invention is different from the above-described mere "immuno-enhancing activity" previously found by the present inventor. Generally, the "immuno-enhancing activity" means an activity in which an enhancement of an immune activity is observed by administering an active ingredient having such an activity in comparison with a state before the administration, that is, an activity of enhancing an immune activity per se. The state before the administration may be a state in which an immune activity is natural or lowered by stress. In contrast, the "activity of promoting a recovery from stress" in the present invention is an activity which promotes the recovery of an immune activity during a period of an immune activity convalescence, as described above, that is, an activity of enhancing the speed of recovery of an immune activity. When the pharmaceutical composition of the present invention for promoting a recovery from stress is administered, the speed of recovery of an immune activity is increased in comparison with the case in which the pharmaceutical composition of the present invention for promoting a recovery from stress is not administered.

Further, in the "immuno-enhancing activity", enhancement of an immune activity is directly observed when administering an active ingredient having such an activity. In contrast, when administering the active ingredient of the pharmaceutical composition of the present invention for promoting a recovery from stress (i.e., *Tricholoma matsutake*, the hot water extract of *Tricholoma matsutake* or the dried product thereof, or the alkaline solution extract of *Tricholoma matsutake* or the dried product thereof) to a subject animal before a loading of stress, the recovery of an immune activity is promoted during a period of an immune activity convalescence, even if the active ingredient is not administered during a loading of stress and during a period of an immune activity convalescence. With respect to the point, the "activity of promoting a recovery from stress" in the present invention is different from the "immuno-enhancing activity".

The formulation of the pharmaceutical composition of the present invention for promoting a recovery from stress is not particularly limited to, but may be, for example, oral medicines, such as powders, fine particles, granules, tablets, capsules, suspensions, emulsions, syrups, extracts or pills, or parenteral medicines, such as injections, liquids for external use, ointments, suppositories, creams for topical application, or eye lotions.

The oral medicines may be prepared by an ordinary method using, for example, fillers, binders, disintegrating agents, surfactants, lubricants, flowability-enhancers, diluting agents, preservatives, coloring agents, perfumes, tasting agents, stabilizers, humectants, antiseptics, antioxidants or the like, such as gelatin, sodium alginate, starch, corn starch, saccharose, lactose, glucose, mannitol, carboxylmethylcellulose, dextrin, polyvinyl pyrrolidone, crystalline cellulose, soybean lecithin, sucrose, fatty acid esters, talc, magnesium stearate, polyethylene glycol, magnesium silicate, silicic anhydride, or synthetic aluminum silicate.

The parenteral administration may be, for example, an injection such as a subcutaneous or intravenous injection, or a per rectum administration. Of the parenteral formulations, an injection is preferably used.

When the injections are prepared, for example, watersoluble solvents, such as physiological saline or Ringer's solution, water-insoluble solvents, such as plant oil or fatty acid ester, agents for rendering isotonic, such as glucose or sodium chloride, solubilizing agents, stabilizing agents, antiseptics, suspending agents, or emulsifying agents may be optionally used, in addition to the active ingredient.

The pharmaceutical composition of the present invention for promoting a recovery from stress may be administered in the form of a sustained release preparation using sustained release polymers. For example, the pharmaceutical composition of the present invention for promoting a recovery from stress may be incorporated to a pellet made of ethylenevinyl acetate polymers, and the pellet may be surgically implanted in a tissue to be treated.

The pharmaceutical composition of the present invention for promoting a recovery from stress may contain *Tricholoma matsutake*, the hot water extract of *Tricholoma matsutake* or the dried product thereof, or the alkaline solution extract of *Tricholoma matsutake* or the dried product thereof in an amount of, but is by no means limited to, 0.01 to 99% by weight, preferably 0.1 to 80% by weight.

A dose of the pharmaceutical composition of the present invention for promoting a recovery from stress is not particularly limited, but may be determined dependent upon the kind of disease, the age, sex, body weight, or symptoms of the subject, a method of administration, or the like. The immuno-enhancing composition of the present invention may be orally or parenterally administered.

The pharmaceutical composition of the present invention for promoting a recovery from stress may be administered as a medicament or in various forms, for example, eatable or drinkable products, such as functional foods or health foods, or feeds. Further, the pharmaceutical composition of the present invention for promoting a recovery from stress may be administered in the form of an oral hygienic composition, which is temporarily kept in the mouth, but then spat out, retaining almost none of the components, for example, a dentifrice, a mouthwash agent, a chewing gum, or a collutorium, or in the form of an inhalation drawn in through the nose. For example, *Tricholoma matsutake,* the hot water extract of *Tricholoma matsutake* or the dried product thereof, or the alkaline solution extract of *Tricholoma matsutake* or the dried product thereof may be added to a desired food including a drink, a feed, a dentifrice, a mouthwash agent, a chewing gum, a collutorium, or the like as an additive, such as a food additive.

The *Tricholoma matsutake* strain FERM BP-7304 was deposited in the International Patent Organism Depositary National Institute of Advanced Industrial Science and Technology [(Former Name) National Institute of Bioscience and Human-Technology Agency of Industrial Science and Technology (Address: AIST Tsukuba Central 6, 1-1, Higashi 1-chome Tukuba-shi, Ibaraki-ken 305-8566 Japan)] on September 14, 2000. The *Tricholoma matsutake* strain FERM BP-7304 was established as a strain by culturing a piece of fruit body from a *Tricholoma matsutake* strain CM6271 collected in Kameoka-shi, Kyoto, Japan and then culturing it in vitro, and is maintained in the Biomedical Research Laboratories, Kureha Chemical Industry Co. Ltd.

The macroscopical observation of a fruit body of the *Tricholoma matsutake* strain FERM BP-7304 of the present invention is identical with that of a *Tricholoma matsutake* fruit body described in Rokuya Imaseki and Tsuguo Hongo, "Gensyoku Nihon Shinkinrui Zukan (Colored Illustrations of Mushrooms of Japan) (I)", Hoiku-sha (Osaka, Japan), 1987, plate 15 and page 77. The *Tricholoma matsutake* strain FERM BP-7304 of the present invention can be subcultivated or maintained on a MATSUTAKE medium. A large-scale cultivation of mycelia of the *Tricholoma matsutake* strain FERM BP-7304 can be carried out by inoculating the strain into a liquid medium and then performing, for example, a static culture, a shake culture, or a tank culture.

When mycelia of the *Tricholoma matsutake* strain FERM BP-7304 of the present invention are inoculated on a MATSUTAKE medium, white hyphae grow radially and thickly, and form large colonies. According to an observation by a scanning electron microscope, a large number of branched mycelia having a diameter of 1 to 2 μm exist and projections having a height of approximately 2 to 3 μm are observed at the side of mycelia. In this connection, the *Tricholoma matsutake* strain FERM BP-7304 can be subcultured or cultured, generally in the form of mycelia, but sometimes in the form of fruit bodies.

Mycological features of the *Tricholoma matsutake* strain FERM BP-7304 of the present invention will be explained hereinafter.

(1) Cultural and morphological features on malt extract agar medium

When the *Tricholoma matsutake* strain FERM BP-7304 of the present invention is inoculated on a malt extract agar medium, white hyphae grow radially and thickly, and form colonies. A diameter of the colony after 30 days from the inoculation is approximately 4 cm.

(2) Cultural and morphological features on potato and glucose agar medium, Czapek's agar medium, sabouraud agar medium, oatmeal agar medium, synthetic mucor agar medium, and medium for assaying phenol oxidase reaction

When the *Tricholoma matsutake* strain FERM BP-7304 of the present invention is inoculated on a potato and glucose agar medium, a Czapek's agar medium, a sabouraud agar medium, an oatmeal agar medium, a synthetic mucor agar medium, or a medium for assaying a phenol oxidase reaction, a growth of hyphae is not observed after a month from the inoculation.

(3) Cultural and morphological features on YpSs agar medium

The *Tricholoma matsutake* strain FERM BP-7304 of the present invention grows as a mat-like mycelia having a white gloss on a YpSs agar medium. A growth area after 30 days from the inoculation is approximately 5 mm in radius.

(4) Cultural and morphological features on glucose and dry yeast agar medium

The *Tricholoma matsutake* strain FERM BP-7304 of the present invention grows as a mat-like mycelia having white gloss on a glucose and dry yeast agar medium. A growth distance after 30 days from the inoculation is approximately 2 mm.

(5) Optimal growth temperature and range of temperature

After 100 mL-conical flasks each containing 10 mL of sterile medium (3% glucose and 0.3% yeast extract, pH 7.0) were inoculated with approximately 2 mg of a piece of the *Tricholoma matsutake* strain FERM BP-7304 mycelia of the present invention, cultivation was performed at various temperatures from 5 to 35 °C. After the cultivation for 28 days, mycelia were taken from the flasks, washed thoroughly with distilled water, and dried, and then each weight of mycelia was measured. As a result, the weight of mycelia increased linearly at a range of 5 to 15 °C, and gently at a range of 15 to 25 °C. Mycelia did not grow at 27.5 °C or more. The optimal growth temperature is 15 to 25 °C.

(6) Optimal growth pH and range of pH

A growth pH value was examined by preparing various media having a pH in a range of 3.0 to 8.0. These media were prepared by adjusting a pH of a liquid medium (3% glucose and 0.3% yeast extract) with 1 mol/L HCL or 1 mol/L potassium hydroxide. Each medium was sterilized through a filter, and then 10 mL of the sterile medium was poured into a 100 mL-conical flask (previously sterilized). After approximately 2 mg of a piece of the *Tricholoma matsutake* strain FERM BP-7304 mycelia of the present invention was inoculated, cultivation was performed at 22 °C. After the cultivation for 28 days, mycelia were taken from the flasks, washed thoroughly with distilled water, and dried, and then each weight of mycelia was measured. As a result, the limit for growth of mycelia was in a range of a pH 3.0 to 7.0. the optimal growth pH was 4.0 to 6.0.

(7) Formation of confront line by confrontation culture

A block (approximately 3 mm × 3 mm × 3 mm) of the *Tricholoma matsutake* strain FERM BP-7304 of the present invention and each block (approximately 3 mm × 3 mm × 3 mm) of 13 kinds of *Tricholoma matsutake* strains listed in Table 1 described below were inoculated with a space of approximately 2 cm therebetween on a MATSUTAKE medium. After a cultivation was carried out at 22 °C for 3 weeks, it was observed whether or not a zone appeared at the boundary between two colonies.

As a result, the *Tricholoma matsutake* strain FERM BP-7304 of the present invention did not form a clear zone with respect to the 13 kinds of *Tricholoma matsutake* strains listed in Table 1. In this connection, it is considered that *Tricholoma matsutake* does not form a zone in a confrontation culture. Among the 13 kinds of *Tricholoma matsutake* strains listed in Table 1, no combinations formed a clear zone.

(8) Auxotrophy

After a 100 mL-conical flask containing 10 mL of a sterile synthetic medium for mycorrhizae (Ohta et al., Trans. Mycol. Soc. Jpn., 31, 323, 1990) was inoculated with approximately 2 mg of a piece of the *Tricholoma matsutake* strain FERM BP-7304 mycelia of the present invention, cultivation was performed at 22 °C. After the cultivation for 42 days, mycelia were taken from the flask, washed thoroughly with distilled water, and dried, and then the weight of mycelia was measured to obtain 441 mg of mycelia.

Each medium in which one of 28 kinds of sugar-related substances was substituted for glucose as a carbon (C) source in the synthetic medium for mycorrhizae was inoculated with the *Tricholoma matsutake* strain FERM BP-7304 of the present invention, and cultivation was performed. After the cultivation, each weight of mycelia was measured.

As a result, an order of the sugar-related substances from that which achieved the heaviest weight to that which achieved the lightest weight was as follows:

wheat starch > corn starch > dextrin > methyl-β-glucoside > cellobiose >mannose > fructose > arabinose > sorbitol > glucose > lactose > glycogen > mannitol > ribose > maltose > trehalose > galactose > raffinose > melibiose > N-acetylglucosamine.

In this connection, mycelia did not grow in each medium containing cellulose, dulcitol, sucrose, xylose, methyl-α-glucoside, inulin, inositol, or sorbose.

Further, each medium in which one of 15 kinds of nitrogen-related substances was substituted for ammonium tartrate as a nitrogen (N) source in the synthetic medium for mycorrhizae was inoculated with the *Tricholoma matsutake* strain FERM BP-7304 of the present invention, and cultivation was performed. After the cultivation, each weight of mycelia was measured.

As a result, an order of the nitrogen-related substances from that which achieved the heaviest weight to that which achieved the lightest weight was as follows:

corn steep liquor > soybean peptone > milk peptone > ammonium nitrate > ammonium sulfate > ammonium tartrate > ammonium carbonate > asparagine > ammonium phosphate > ammonium chloride > sodium nitrate > meat extract > yeast extract > casamino acids > chlorella > tryptone > potassium nitrate.

Furthermore, each medium in which one component among minerals and vitamins in the above synthetic medium was removed was inoculated with the *Tricholoma matsutake* strain FERM BP-7304 of the present invention, and cultivation was performed. After the cultivation, each weight of mycelia was measured.

As a result, a deficiency of any one of calcium chloride dihydrate, manganese sulfate (II) pentahydrate, zinc sulfate heptahydrate, cobalt sulfate heptahydrate, copper sulphate pentahydrate, nickel sulfate hexahydrate, thiamin hydrochloride, nicotinic acid, folic acid, biotin, pyridoxine hydrochloride, carnitine chloride, adenine sulfate dihydrate, or choline hydrochloride did not affect the weight of mycelia. In contrast, when any one of magnesium sulfate heptahydrate, iron chloride (II), or sodium dihydrogen phosphate was removed, the weight of mycelia was remarkably lowered. From the results, it is considered that magnesium, iron, phosphorus, and potassium are essential for the growth of the *Tricholoma matsutake* strain FERM BP-7304 of the present invention.

(9) Base composition of DNA (GC content)

The GC content of the *Tricholoma matsutake* strain FERM BP-7304 of the present invention is 49.9% (see Analytic Example 2 described below).

(10) DNA patterns generated by a RAPD method

With respect to each DNA pattern generated by a RAPD (random amplified polymorphic DNA) method using any one of six kinds of primers (10mer; the actual base sequences are shown in Analytical Example 1 described below) for PCR (polymerase chain reaction), the *Tricholoma matsutake* strain FERM BP-7304 of the present invention was compared with 44 kinds of *Tricholoma matsutake* strains (the actual strains are shown in Analytical Example 1 described below). As a result, it was confirmed that DNA patterns of the *Tricholoma matsutake* strain FERM BP-7304 of the present invention differed from those of 44 kinds of *Tricholoma matsutake* strains.

EXAMPLES

The present invention now will be further illustrated by, but is by no means limited to, the following Examples.

Example 1: Preparation of dry powder of *Tricholoma matsutake* strain FERM BP-7304

After 500 mL-conical flasks (10 flasks) each containing 100 mL of sterile medium (3% glucose and 0.3% yeast extract, pH 6.0) were inoculated with mycelia of *Tricholoma matsutake* strain FERM BP-7304, cultivation was performed in a shaking incubator (250 rpm) at 22 °C for 4 weeks. The resulting broth was filtered with a filter cloth to separate mycelia. The mycelia were washed with distilled water. After having been frozen at -60 °C, the mycelia were lyophilized using a lyophilizer (MINIFAST MOD. DO. 5; Edwards) to obtain 10.1 g of dried mycelia. The resulting dried mycelia were crushed by a homoblender (Wonder Blender; Osaka Chemical Co., Ltd.) to obtain 9.8 g of dry powder.

Example 2: Preparation of dry powder of hot water extract and alkaline solution extract of *Tricholoma matsutake* strain FERM BP-7304

After 15 g of dry powder of *Tricholoma matsutake* strain FERM BP-7304 mycelia prepared in a similar manner as that described in Example 1 and 600 mL of purified water were charged in a 1 L-beaker, an extraction treatment was performed in a water bath at 93 to 98 °C for 3 hours while stirring. After the extraction was completed, the whole was cooled to room temperature and centrifuged at 12,000 rpm for 20 minutes to obtain a supernatant.

To the remaining pellets, 300 mL of purified water was added, and the same procedures as above were performed. These procedures were repeated three times. Supernatants obtained by each procedure and the supernatant previously obtained were combined. The resulting mixture was put into a dialysis membrane with a fractioning molecular weight of 3500 (Spetra/Por 3 Membrane), and dialyzed in flowing tap water for 2 days. The inner part of the dialyzate was concentrated by a rotary evaporator and lyophilized to obtain 3.2 g of dry powder of a hot water extract.

To the pellets remaining after the hot water extraction treatment, 400 mL of 0.5 mol/L sodium hydroxide solution was added. Extraction was performed at 25 °C for 1 hour while stirring. After extraction, the whole was centrifuged (12,000 rpm, 20 minutes) to obtain a supernatant.

To the remaining pellets, 400 mL of 1.0 mol/L sodium hydroxide solution was added. The same procedures as above were performed to obtain a supernatant. Two supernatants were combined, and the pH of the supernatant was adjusted to 7.0 by adding 1.0 mol/L HCl thereto. The whole was put into a dialysis membrane (Spetra/Por 3 Membrane, fractioning molecular weight = 3500), and dialyzed in flowing tap water for 2 days. The inner part of the dialyzate was concentrated by a rotary evaporator and lyophilized to obtain 5.1 g of dry powder of an alkaline solution extract.

Example 3: Preparation of dry powder of *Tricholoma matsutake* fruit bodies from Iwate Prefecture

After 250 g of fruit bodies of commercially available *Tricholoma matsutake* harvested in Iwate Prefecture (purchased at Kuroshio market; Kita-Shinjuku, Shinjuku-ku, Tokyo) were lyophilized using a lyophilizer (MINIFAST MOD. DO. 5; Edwards) to remove water, the lyophilized fruit bodies were crushed by a homoblender (Wonder Blender; Osaka Chemical Co., Ltd.) to obtain 35 g of dry powder.

Example 4: Preparation of dry powder of hot water extract and alkaline solution extract of *Tricholoma matsutake* from Iwate Prefecture

After 20 g of dry powder of commercially available *Tricholoma matsutake* fruit bodies from Iwate Prefecture prepared in a similar manner as that described in Example 3 and 800 mL of purified water were charged in 1 L-beaker, an extraction treatment was performed in a water bath at 93 to 98 °C for 3 hours while stirring. After the extraction was completed, the whole was cooled to room temperature and centrifuged at 12,000 rpm for 20 minutes to obtain a supernatant.

To the remaining pellets, 500 mL of purified water was added, and the same procedures as above were performed. These procedures were repeated three times. Supernatants obtained by each procedure and the supernatant previously obtained were combined. The resulting mixture was put into a dialysis membrane with a fractioning molecular weight of 3500 (Spetra/Por 3 Membrane), and dialyzed in flowing tap water for 2 days. The inner part of the dialyzate was concentrated by a rotary evaporator and lyophilized to obtain 1.0 g of dry powder.

To the pellets remaining after the hot water extraction treatment, 500 mL of 0.5 mol/L sodium hydroxide solution was added. Extraction was performed at 25 °C for 1 hour while stirring. After extraction, the whole was centrifuged (12,000 rpm, 20 minutes) to obtain a supernatant.

To the remaining pellets, 500 mL of 1.0 mol/L sodium hydroxide solution was added. The same procedures as above were performed to obtain a supernatant. Two supernatants were combined, and the pH of the supernatant was adjusted to 7.0 by adding 1.0 mol/L HCl thereto. The whole was put into a dialysis membrane (Spetra/Por 3 Membrane, fractioning molecular weight = 3500), and dialyzed in flowing tap water for 2 days. The inner part of the dialyzate was concentrated by a rotary evaporator and lyophilized to obtain 5.1 g of dry powder of an alkaline solution extract.

Example 5: Preparation of dry powder of hot water extract of *Tricholoma matsutake* fruit bodies from Nagano Prefecture

The procedures described in Example 4 were repeated, except that *Tricholoma matsutake* fruit bodies harvested in Nagano Prefecture were used instead of those harvested in Iwate Prefecture, to obtain 1.5 g of dry powder of a hot water extract of *Tricholoma matsutake* fruit bodies.

Comparative Example 1: Preparation of dry powder of hot water extract of *Agaricus blazei* fruit bodies

The procedures described in Example 4 were repeated, except that commercially available *Agaricus blazei* fruit bodies were used instead of *Tricholoma matsutake* fruit bodies harvested in Iwate Prefecture, to obtain 3.6 g of dry powder of a hot water extract of *Agaricus blazei* fruit bodies.

Comparative Example 2: Preparation of dry powder of hot water extract of *Ganoderma lucidum (Fr.) Karst* fruit bodies

The procedures described in Example 4 were repeated, except that commercially available *Ganoderma lucidum (Fr.) Karst* fruit bodies were used instead of *Tricholoma matsutake* fruit bodies harvested in Iwate Prefecture, to obtain 2.4 g of dry powder of a hot water extract of *Ganoderma lucidum (Fr.) Karst* fruit bodies.

Comparative Example 3: Preparation of dry powder of hot water extract of *Lentinus edodes (Berk.) Sing* . fruit bodies

The procedures described in Example 4 were repeated, except that commercially available *Lentinus edodes (Berk.) Sing.* fruit bodies were used instead of *Tricholoma matsutake* fruit bodies harvested in Iwate Prefecture, to obtain 1.4 g of dry powder of a hot water extract of *Lentinus edodes (Berk.) Sing.* fruit bodies.

Analytical Example 1: Identification of *Tricholoma matsutake* strain FERM BP-7304 by RAPD method

In the present analytical example, DNA patterns which were amplified by a random amplified polymorphic DNA (RAPD) method using DNA of *Tricholoma matsutake* strain FERM BP-7304 according to the present invention and any one of six kinds of primers (10mer) for a polymerase chain reaction (PCR) were compared with those of plural known *Tricholoma matsutake* strains.

As the known strains for comparison, 13 kinds of *Tricholoma matsutake* strains listed in Table 1 were used. Dried mycelia were obtained, and then the resulting mycelia were crushed to obtain dry powder, in a similar manner as that described in Example 1. Yields of mycelia of *Tricholoma matsutake* strains (weight of dried mycelia per 100 mL of medium; an average of 10 samples) and origins (facilities in which the strains were established) of *Tricholoma matsutake* strains are shown in Table 1.

Table 1

| No. | Strain | Yield (g) | Origin (Facility) |
|---|---|---|---|
| 1 | IFO 6915 | 0.67 | Institute for Fermentation, Osaka |
| 2 | IFO 6925 | 0.50 | Institute for Fermentation, Osaka |
| 3 | IFO 6930 | 0.72 | Institute for Fermentation, Osaka |
| 4 | IFO 6935 | 0.65 | Institute for Fermentation, Osaka |
| 5 | CM 627-2 | 0.79 | Kureha Chemical Industry Co. Ltd |
| 6 | CM 627-4 | 0.80 | Kureha Chemical Industry Co. Ltd |
| 7 | IFO 30604 | 0.49 | Institute for Fermentation, Osaka |
| 8 | IFO 30605 | 0.71 | Institute for Fermentation, Osaka |
| 9 | IFO 30606 | 0.35 | Institute for Fermentation, Osaka |
| 10 | MAFF 460039 | 0.56 | National Institute of Agrobiological Science; Ministry of Agriculture, Forestry and Fisheries of Japan |
| 11 | KT 001 | 0.68 | Kureha Chemical Industry Co. Ltd |
| 12 | IFO 6920 | 0.72 | Institute for Fermentation, Osaka |
| 13 | IFO 6933 | 0.56 | Institute for Fermentation, Osaka |

As the primers for PCR, the following primers:

```
primer RAPD1 (SEQ ID NO: 1; TGGTCACCGA),
```

```
primer RAPD2 (SEQ ID NO: 2; AGCGCCATTG),
```

```
primer RAPD3 (SEQ ID NO: 3; TTCGAGCCAG),

primer RAPD4 (SEQ ID NO: 4; TGCGTGCTTG),

primer RAPD5 (SEQ ID NO: 5; GACTAGCCTC),
```

and

```
primer RAPD6 (SEQ ID NO: 6; CTCACCGTCC)
```

were prepared by a chemical synthesis method.

The DNAs used in the RAPD method were prepared using a commercially available DNA preparation kit (Dneasy Plant Mini Kit; QIAGEN) by a method similar to that described in an attached manual [Dneasy Plant Mini Handbook for DNA isolation from plant tissue (March, 1999, QIAGEN, K. K., Tokyo)] as described below.

After removing water roughly, each mycelia (approximately 100 mg) of *Tricholoma matsutake* strains was put into a tube, frozen with liquid nitrogen, and kept at -80 °C until using. Each tube containing mycelia was thawed, and then an AP1 buffer (400 μL) and an RNase A stock solution (4 μL) were added to the tube. The whole was thoroughly mixed by a vortex mixer. The tube was incubated in a water bath at 65 °C for 10 minutes. The tube was inverted several times to mix the contents during the incubation. After the incubation, an AP2 buffer (130 μL) was added to the mixture, and then the whole was allowed to stand on ice for 5 minutes. The contents in the tube were applied to a column (QIAshredder spin column). The column was put into a 2 mL-test tube and centrifuged at 15000 rpm for 2 minutes. After the centrifugation, a solution which had passed through the column and had gathered at the bottom of the tube (passage liquid) was carefully transferred to another test tube, so as not to touch cell pellets at the bottom of the tube. A volume of the passage liquid was measured.

An equal volume of 100% ethanol and a half volume of a buffer AP3, with respect to the passage liquid, were added to the test tube and mixed thoroughly. After 650 μL of the mixture was applied to another column (DNeasy mini spin column), the column was put into a 2 mL-test tube and centrifuged at 8000 rpm for 1 minute. After the centrifugation, an AW buffer (500 μL) was applied to the column. The column was put into the 2 mL-test tube and centrifuged at 8000 rpm for 1 minute, and then a solution collected at the bottom of the test tube was discarded. After the AW buffer (500 μL) was applied to the column, the column was put into the 2 mL-test tube and centrifuged at 15000 rpm for 2 minutes, and then a solution collected at the bottom of the test tube was discarded. The washed column was put into a new test tube. After an AE buffer (100 μL) kept at 65 °C was applied to the washed column, the column was allowed to stand at room temperature for 5 minutes, and centrifuged at 8000 rpm for 1 minute to collect a DNA solution at the bottom of the test tube. The similar procedures were repeated once again to collect the DNA solution (approximately 200 μL in total). A portion (20 μL) of the resulting DNA solution was electrophoresed on a 1% agarose gel to determine an amount of DNA recovered and a purity of the DNA.

A PCR was carried out in a reaction solution (a total volume = 25 μL) shown in Table 2. In the PCR, a cycle consisting of treatments at 94°C for 30 seconds (a denaturing step), 36°C for 1 minute (an annealing step), and 72°C for 2 minutes (an elongation step) was repeated 45 times. In this connection, a preliminary denaturing step for 2 minutes was performed before the first cycle, and a final elongation step for 2 minutes was performed after the 45th cycle. The reaction solution shown in Table 2 was prepared by mixing an Ex Taq™ buffer, TaKaRa Ex Taq™, and a Taq Start antibody, adding distilled water, a 10×Ex Taq™ buffer, dNTP, and a primer to the mixture after 2 to 3 seconds from the mixing treatment, and finally adding a DNA solution.

Table 2

| Reaction solution (per a tube) | |
|---|---|
| Ex Taq™ buffer (Takara Shuzo) | 0.8 μL |
| TaKaRa Ex Taq™ (Takara Shuzo) | 0.2 μL |
| Taq Start antibody (Takara Shuzo) | 0.2 μL |
| Distilled water | 8.3 μL |
| 10×Ex Taq™ buffer (Takara Shuzo) | 2.5 μL |
| dNTP | 2.0 μL |

Table 2   (continued)

| Reaction solution (per a tube) | |
| --- | --- |
| Primer | 1.0 μL |
| DNA | 10 μL |

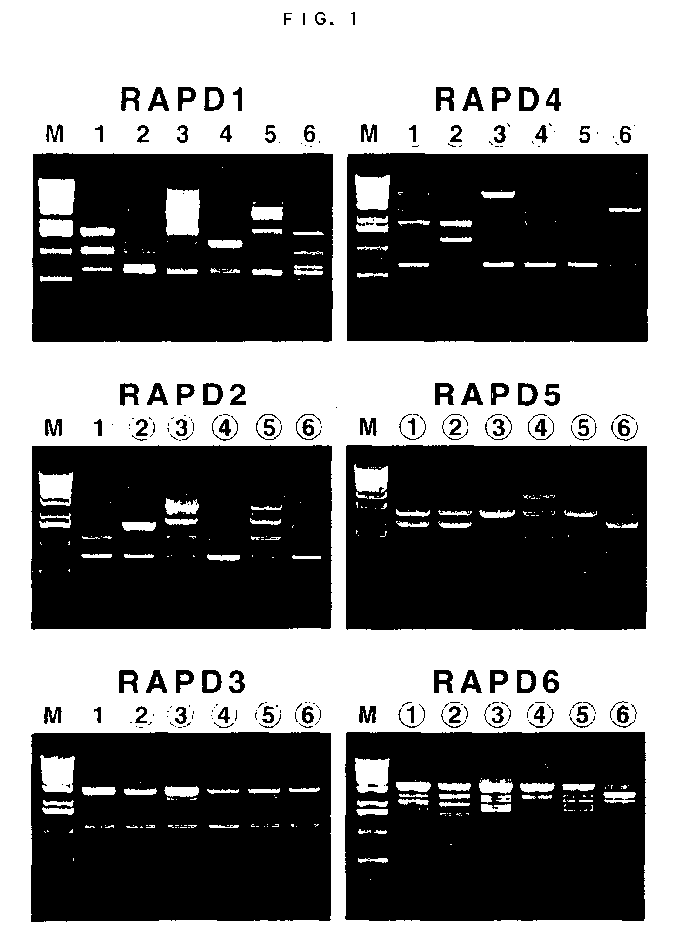

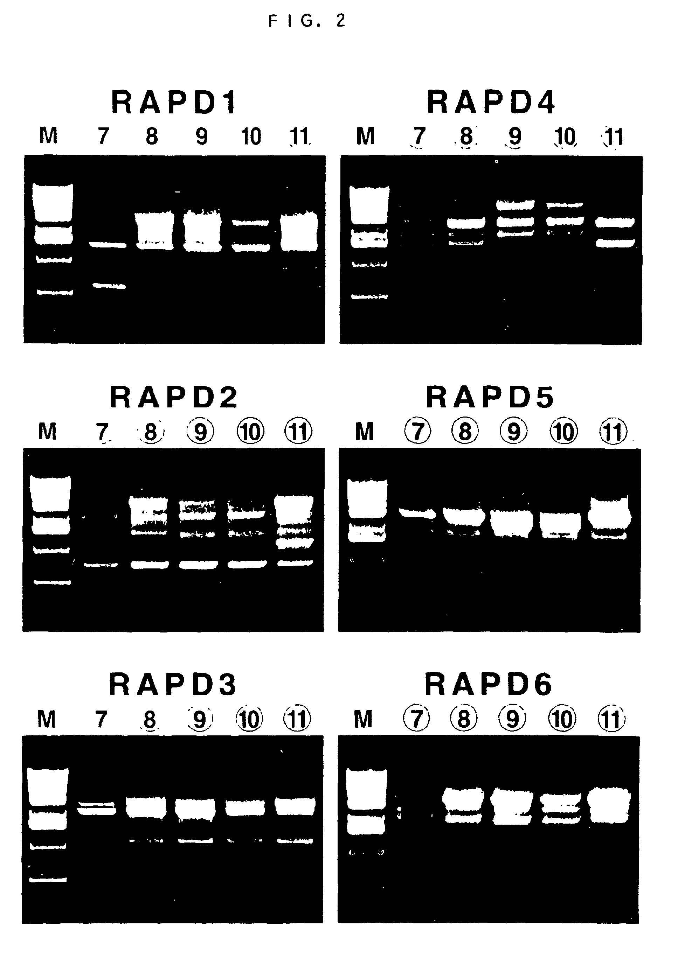

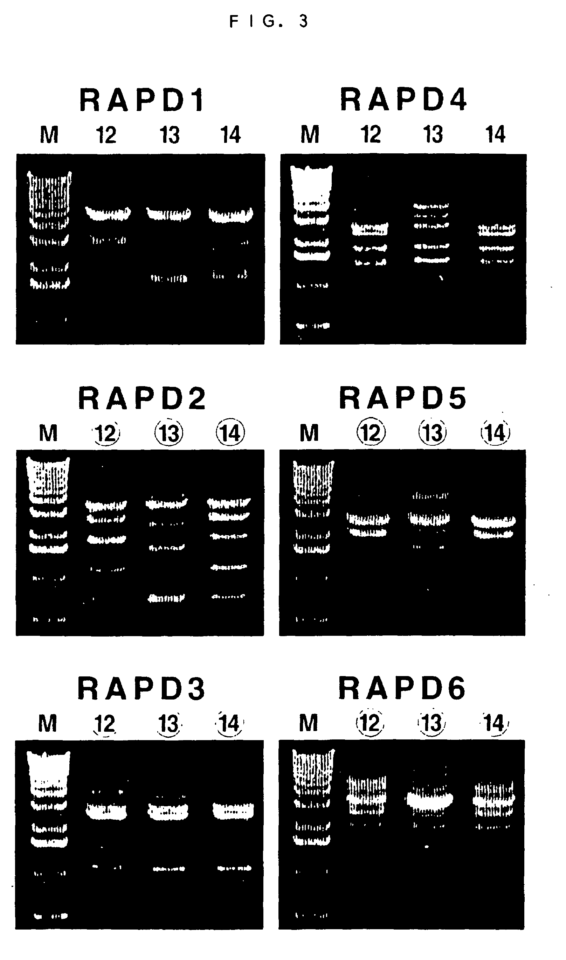

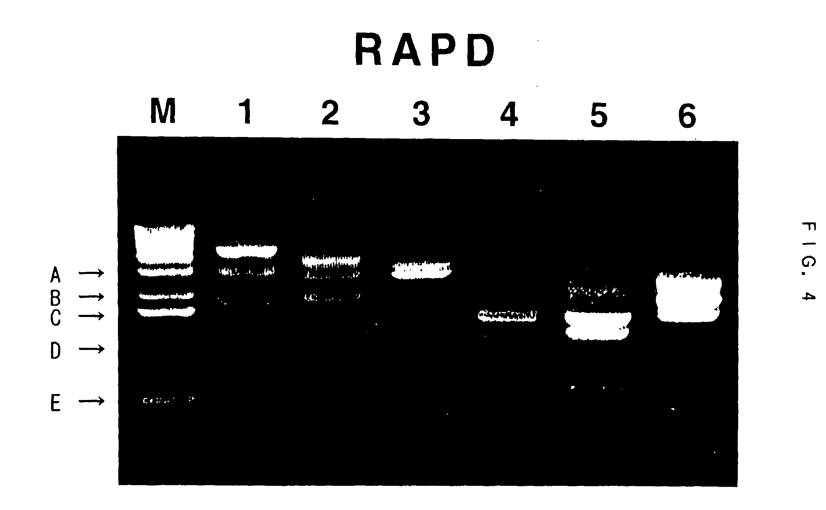

DNA patterns obtained by separating the resulting PCR products by an agarose gel electrophoresis are shown in Figs. 1 to 4. Fig. 1 shows DNA patterns of comparative strains of Nos. 1 to 6 in Table 1; Fig. 2 shows DNA patterns of comparative strains of Nos. 7 to 11 in Table 1; Fig. 3 shows DNA patterns of comparative strains of Nos. 12 to 13 in Table 1 and *Tricholoma matsutake* strain FERM BP-7304 of the present invention; and Fig. 4 shows DNA patterns of *Tricholoma matsutake* strain FERM BP-7304 of the present invention.

Circled numbers "1" to "6" in Fig. 1, circled numbers "7" to "11" in Fig. 2, and circled numbers "12" to "13" in Fig. 3 correspond to the strain Nos. 1 to 13 in Table 1, respectively. For example, lanes represented by the circled number "1" in Fig. 1 show results of the strain No. 1 (IFO 6915) in Table 1. Further, lanes represented by the circled number "14" in Fig. 3 show results of *Tricholoma matsutake* strain FERM BP-7304 of the present invention.

Words "RAPD1" to "RAPD6" in Figs. 1 to 3 mean primers RAPD1 to RAPD6, respectively.

Lanes 1 to 6 in Fig. 4 show results of *Tricholoma matsutake* strain FERM BP-7304 of the present invention obtained using primers RAPD1 to RAPD6, respectively.

Further, a symbol "M" in Figs. 1 to 4 means a DNA molecular weight marker. A single DNA molecular weight marker (1kb DNA ladder, catalog No. 15415-018, Life Technologies, GIBCO-BRL, USA) was used in the electrophoresis. In Fig. 4, a band represented by an arrow A shows a DNA fragment of 4072 bp; a band represented by an arrow B shows a DNA fragment of 3054 bp; a band represented by an arrow C shows a stack of DNA fragments of 2036 bp and 1636 bp; a band represented by an arrow D shows a DNA fragment of 1018 bp; and a band represented by an arrow E shows a stack of DNA fragments of 517 bp, 506 bp, 396 bp, 344 bp, and 298 bp.

As shown in Figs. 1 to 4, DNA patterns of *Tricholoma matsutake* strain FERM BP-7304 were different from those of 13 kinds of *Tricholoma matsutake* strains for comparison listed in Table 1.

Further, although DNA patterns are not actually shown, DNA patterns of *Tricholoma matsutake* strain FERM BP-7304 were different from those of 31 kinds of *Tricholoma matsutake* strains other than 13 kinds of comparative strains listed in Table 1. The 31 kinds of strains were

MAFF 460031, MAFF 460033, MAFF 460034, MAFF 460035, MAFF 460036, MAFF 460037, MAFF 460038, MAFF 460040, MAFF 460041, MAFF 460042, MAFF 460046, MAFF 460050, and MAFF 460096 (National Institute of Agrobiological Science; Ministry of Agriculture, Forestry and Fisheries of Japan);

CM 627-3, CM 627-5, CM 627-6, and CM 627-7(Kureha Chemical Industry Co. Ltd); and

IFO 6929, IFO 6931, IFO 6932, IFO 6934, IFO 6916, IFO 6917, IFO 6918, IFO 6919, IFO 6921, IFO 6922, IFO 6923, IFO 6924, IFO 6926, and IFO 6928 (Institute for Fermentation, Osaka).

Analytical Example 2: GC content of *Tricholoma matsutake* strain FERM BP-7304

After 20 mL of a phosphate-buffered saline (PBS) solution (pH7.4) containing 1 mg/mL proteinase K (Merck, Germany) was added to a 50 mL-conical flask containing mycelia (wet-base weight = 1.0 g) of *Tricholoma matsutake* strain FERM BP-7304 prepared in a similar manner as that described in Example 1, the whole was reacted at 65 °C for 2 hours while occasionally shaking. The reaction mixture was heated at 100 °C for 10 minutes to inactivate the enzyme, and cooled to 37 °C. An appropriate amount (approximately 1 mg) of Zymolyase (Seikagaku Corp.) was added to the reaction mixture, and the whole was reacted at 37 °C for 4 hours. After 40 mL of a Tris-SDS (sodium dodecyl sulfate) buffer was added, mycelia was lysed at 60 °C to prepare a DNA extract.

An equal volume of phenol was added to the DNA extract. After mixing, the mixture was centrifuged to remove a phenol layer. Crude DNA was recovered as pellets from the resulting supernatant by an ethanol precipitation method, and washed with 70 to 90% ethanol. The resulting DNA pellets were dissolved in 5 mL of a citrate buffer, and then an RNase treatment was performed. A small amount of phenol was added to the RNase-treated solution. After mixing, the mixture was centrifuged to remove a phenol layer. Crude DNA was recovered from the resulting supernatant by the ethanol precipitation method, and washed with 70 to 90% ethanol. After the second RNase treatment was performed, 0.5 mL of an EDTA(ethylenediaminetetraacetic acid)-containing acetate buffer was added. DNA was precipitated by adding propanol and recovered by centrifugation. The resulting DNA was washed with 70 to 90% ethanol, and finally dissolved in 1 mL of a citrate buffer to obtain a purified DNA solution.

A portion (100 μL) of the resulting purified DNA solution was heated at 100 °C for 10 minutes, cooled on ice, and treated with nuclease P1 at 50 °C for 1 hour to obtain nucleotide products. A GC content was measured by a high performance liquid chromatograph (LC-6A; Shimadzu). A GC kit (Yamasa Corporation) was used as a standard, a column YMC-Pack AQ-312 (diameter = 6.0 mm, length = 150 mm) was used as a column, and a 0.2 mol/L ammonium phosphate solution (pH = approximately 4.5) was used as a mobile phase. An amount of injection was 10μL, detection was carried out at a wavelength of 270 nm, and a peak was identified by a retention time method. A modified percentage method was used as a measuring method.

The GC content of *Tricholoma matsutake* strain FERM BP-7304 was 49.9%.

Example for evaluation 1: Evaluation for activity of promoting a recovery from stress

(1) Evaluation for hot water extract and alkaline solution extract of *Tricholoma matsutake* strain FERM BP-7304

In the present example for evaluation, a mixture of the dry powder of a hot water extract of *Tricholoma matsutake* strain FERM BP-7304 prepared in Example 2, and the dry powder of an alkaline solution extract thereof prepared in Example 2 (a ratio of the dry powder of a hot water extract to that of alkaline solution extract = 3.2:5.1) was used as a sample for evaluation. Further, after the sample for evaluation was orally administered to mice for 4 weeks, restraint stress was loaded for 18 hours, and then a natural killer (NK) cell activity was measured after a release of the stress to examine the effects of the sample.

More particularly, an aqueous solution of the sample for evaluation was orally administered to 8-week-old male C57BL/6 mice (purchased from Japan SLC; 5 to 10 mice per a group) at a dose of 50mg/kg/day for 4 weeks in normal breeding cages. The aqueous solution was prepared by mixing the dry powder of a hot water extract of *Tricholoma matsutake* strain FERM BP-7304 and the dry powder of an alkaline solution extract thereof, which were prepared in Example 2, at a ratio of 3.2 : 5.1, and then dissolving the mixed dry powder in distilled water.

After the administration for 4 weeks, mice were transferred from the normal breeding cages to 50 mL-capped polypropylene centrifuge tubes (catalog No. 2341-050; Asahi Techno Glass Corporation) with air vents so that a mouse was confined in a tube. The confined mice could not move in the tubes. The tubes in which mice were confined were placed in the cages and allowed to stand for 18 hours to load the mice with restraint stress. After the stress loading for 18 hours, mice were transferred from the tubes to the breeding cages, and bred under ordinary breeding conditions.

After a predetermined number of days [0 day (immediately after the release), 1 day, 3 days, 5 days, 7 days, and 14 days] had passed from the release of the restraint stress, mice were sacrificed, and a natural killer (NK) cell activity was evaluated by measuring a cytotoxic activity of lymphocytes against an NK-sensitive tumor cell strain YAC-1 in vitro, in accordance with the following procedures.

Spleens and mesenterium lymph nodes were aseptically taken from mice and transferred to a sterile petri dish containing a Hanks balanced salt solution. The lymph nodes were teased with scissors and tweezers, and passed through a mesh to prepare a suspension containing single lymphocyte cells. The cells were washed three times with an RPMI 1640 medium containing a 10% bovine fetal serum which had been heated at 56 °C for 30 minutes. Then, a concentration of cells was adjusted to 5 x 10$^6$/mL with an RPMI 1640 medium containing a 10% bovine fetal serum which had been heated at 56 °C for 30 minutes, 20 mmol/L of 4-(2-hydroxyethyl)-1-piperazine ethanesulfonate, and 30 μg/mL of gentamicin. The resulting cell suspension was used as an effector cell.

The YAC-1 cell used as a target cell was maintained in an RPMI 1640 medium containing a 10% bovine fetal serum which had been heated at 56 °C for 30 minutes, at Biomedical Research Laboratories, Kureha Chemical Industry Co. Ltd. The YAC-1 cells were reacted with radioactive sodium chromate (Amersham Japan) at 37 °C for 20 minutes. Unreacted radioactive sodium chromate was removed by washing three times with an RPMI 1640 medium containing a 10% bovine fetal serum which had been heated at 56 °C for 30 minutes, and a concentration of tumor cells labeled with radioactive chromium was adjusted to 5 x 10$^4$/mL.

0.1 mL of the effector cell suspension or a double-diluted series thereof and 0.1 mL of the suspension of tumor cells labeled with radioactive chromium were put into a test tube, and reacted in a 5% carbon dioxide gas incubator at 37 °C for 4 hours. In this connection, to calculate a "specific lysis" described below, a suspension prepared by putting the tumor cells labeled with radioactive chromium and a medium into a test tube, and a suspension prepared by putting the tumor cells labeled with radioactive chromium and a detergent (Triton; a final concentration = 0.05%) into a test tube were also reacted in a 5% carbon dioxide gas incubator at 37 °C for 4 hours. After the reaction was completed, 1.5 mL of an RPMI 1640 medium containing a 10% bovine fetal serum, which had been heated at 56 °C for 30 minutes, was added to each test tube, and thoroughly mixed by a mixer. The whole was centrifuged at 12,000 rpm for 5 minutes at 4 °C to obtain a supernatant, and a radioactivity was measured by a gamma counter.

A specific lysis (S.L.) was calculated by an equation:

$$[S.L.] = \{(B-Bf)/(B_{max}-B_f)\} \times 100$$

wherein S.L. is a specific lysis (unit = %), B is a radioactivity (unit = Bq) of a supernatant of an experimental group, B$_f$

is a radioactivity (unit = Bq) of a supernatant of a spontaneously releasing group, and $B_{max}$ is a radioactivity (unit = Bq) of a supernatant of a maximum releasing group. The spontaneously releasing group means a group of culturing only the tumor cells labeled with radioactive chromium, and the maximum releasing group means a group of culturing tumor cells labeled with radioactive chromium treated with Triton. The NK cell activity was represented by "Lytic Units 30% (LU30)", that is, a number of cells which kill 30% tumor cells per $10^7$ cells of effector cells.

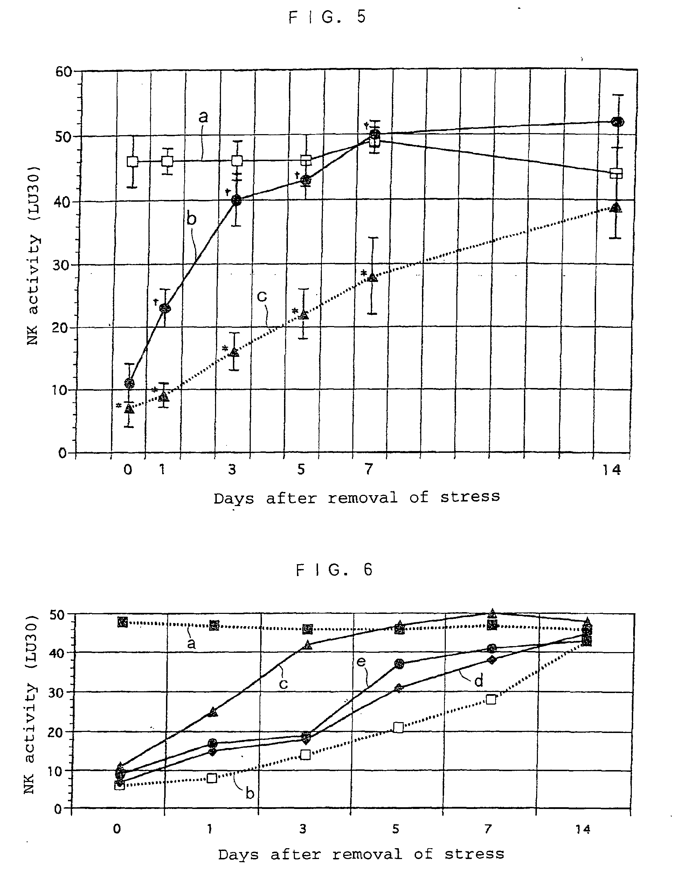

The results are shown in Fig. 5. As a control (a normal group), the above procedures were repeated, except that distilled water was orally administered for 4 weeks, instead of the aqueous solution of the sample for evaluation, and that the restraint stress for 18 hours was not loaded. Further, as a test for comparison, the above procedures were repeated, except that distilled water was orally administered for 4 weeks, instead of the aqueous solution of the sample for evaluation.

In Fig. 5, line a (white square) shows a result of the control (a normal group; without stress), line b (black circle) shows a result when the aqueous solution of the sample for evaluation (the mixture of the dry powder of a hot water extract of *Tricholoma matsutake* strain FERM BP-7304 and the dry powder of an alkaline solution extract thereof, at a ratio of 3.2 : 5.1) according to the present invention was administered, and line c (black triangle) shows a result of the test for comparison (administration of distilled water and stress loading). Further, in Fig. 5, the symbol "+" above black circles in line b means P<0.01 (with respect to a result represented by black triangles in line c), and the symbol "*" above black triangles in line c means P<0.01 (with respect to a result represented by white squares in line a).

As shown in lines b and c in Fig. 5, the NK cell activity is remarkably lowered by loading restraint stress. As shown in line c, after the release of the restraint stress, the NK cell activity was slowly recovered without any treatment. As shown in line c, the recovery of the NK cell activity was significantly promoted by previously administering the mixture of the dry powder of a hot water extract of *Tricholoma matsutake* strain FERM BP-7304 and the dry powder of an alkaline solution extract thereof, at a ratio of 3.2 : 5.1.

(2) Evaluation for dry powder of *Tricholoma matsutake* strain FERM BP-7304, dry powder of *Tricholoma matsutake* fruit bodies from Iwate Prefecture, and mixture of dry powder of a hot water extract of *Tricholoma matsutake* fruit bodies from Iwate Prefecture and dry powder of an alkaline solution extract thereof

The procedures in Example for evaluation 1(1) were repeated except that the dry powder (prepared in Example 1; 50 mg/kg/day) of *Tricholoma matsutake* strain FERM BP-7304, the dry powder (prepared in Example 3; 50 mg/kg/day) of commercially available *Tricholoma matsutake* fruit bodies harvested in Iwate Prefecture, and the mixture (25mg/kg/day) of dry powder (prepared in Example 4) of hot water extract of commercially available *Tricholoma matsutake* fruit bodies harvested in Iwate Prefecture and dry powder (prepared in Example 4) of alkaline solution extract thereof at a ratio of 1.0 : 5.1 were used, instead of the mixture of the dry powder of a hot water extract of *Tricholoma matsutake* strain FERM BP-7304 and the dry powder of an alkaline solution extract thereof at a ratio of 3.2 : 5.1.

The results are shown in Table 3. The symbol "*" in Table 3 means p<0.01 (with respect to administration of distilled water).

Table 3

| | NK cell activity (LU30) | | | | | |
|---|---|---|---|---|---|---|
| | 0day | 1day | 3days | 5days | 7days | 14days |
| Control | 49 | 47 | 48 | 50 | 48 | 48 |
| (without stress loading) | | | | | | |
| Test for comparison | 7 | 9 | 11 | 22 | 30 | 42 |
| (administration of distilled water and stress loading) | | | | | | |
| Example 1 | 10 | 21* | 35* | 46* | 48* | 50 |
| Example 3 | 10 | 20* | 31* | 37* | 49* | 51* |
| Example 4 | 11 | 22* | 37* | 41* | 46* | 48 |

(3) Evaluation for dry powder of *Tricholoma matsutake* strains

The procedures in Example for evaluation 1(1) were repeated except that the dry powder (prepared in Example 1; 50 mg/kg/day) of *Tricholoma matsutake* strain FERM BP-7304, and each dry powder (50 mg/kg/day) of 13 kinds of *Tricholoma matsutake* strains listed in Table 1 and used in Analytical Example 1 were used, instead of the mixture of the dry powder of a hot water extract of *Tricholoma matsutake* strain FERM BP-7304 and the dry powder of an alkaline solution extract thereof at a ratio of 3.2 : 5.1.

The results are shown in Table 4 and Figs. 6 to 9.

The symbol "*" in Table 4 means p<0.01 (with respect to administration of distilled water).

In Figs. 6 to 9, line a (black square) shows a result of the control (without stress), and line b (white square) shows a result of the test for comparison (administration of distilled water and stress loading).

In Fig. 6, line c (black triangle) shows a result of *Tricholoma matsutake* strain FERM BP-7304 of the present invention, line d (black lozenge) shows a result of *Tricholoma matsutake* strain IFO 6915, and line e (black circle) shows a result of *Tricholoma matsutake* strain IFO 6925.

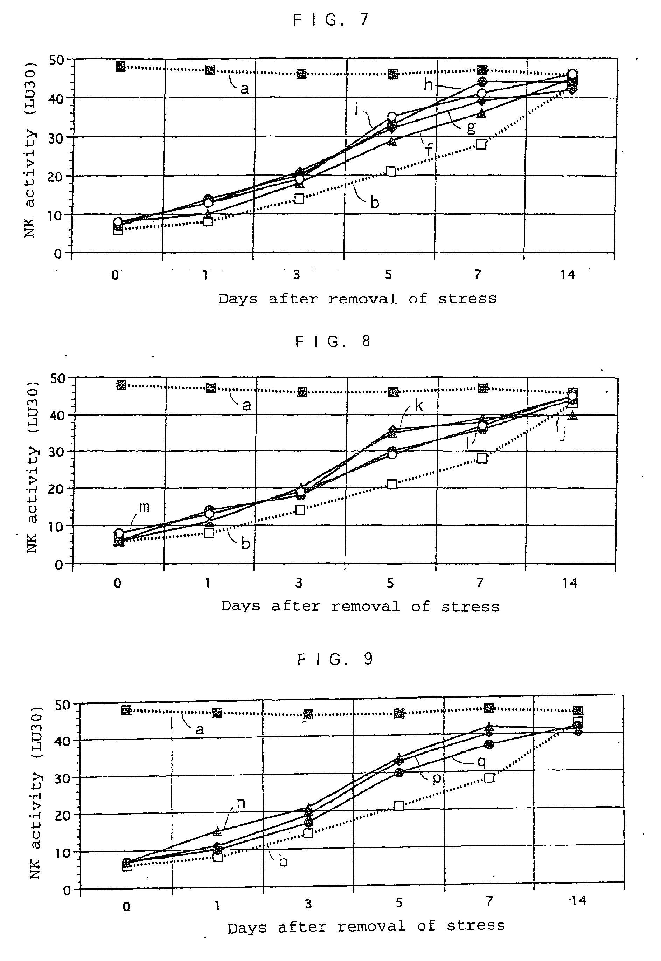

In Fig. 7, line f (black triangle) shows a result of *Tricholoma matsutake* strain IFO 6930, line g (black lozenge) shows a result of *Tricholoma matsutake* strain IFO 6935, line h (black circle) shows a result of *Tricholoma matsutake* strain CM 627-2, and line i (white circle) shows a result of *Tricholoma matsutake* strain CM 627-4.

In Fig. 8, line j (black triangle) shows a result of *Tricholoma matsutake* strain IFO 30604, line k (black lozenge) shows a result of *Tricholoma matsutake* strain IFO 30605, line 1 (black circle) shows a result of *Tricholoma matsutake* strain IFO 30606, and line m (white circle) shows a result of *Tricholoma matsutake* strain MAFF 460039.

In Fig. 9, line n (black triangle) shows a result of *Tricholoma matsutake* strain KT 001, line p (black lozenge) shows a result of *Tricholoma matsutake* strain IFO 6920, and line q (black circle) shows a result of *Tricholoma matsutake* strain IFO 6933.

Table 4

| Strains | NK cell activity (LU30) | | | | | |
|---|---|---|---|---|---|---|
| | 0day | 1day | 3days | 5days | 7days | 14days |
| Control | 48 | 47 | 46 | 46 | 47 | 46 |
| (without stress loading) | | | | | | |
| Test for comparison 6 | | 8 | 14 | 21 | 28 | 43 |
| (administration of distilled water and stress loading) | | | | | | |
| [Examples] | | | | | | |
| FERM BP-7304 | 11 | 25* | 42* | 47* | 50* | 48 |
| IFO 6915 | 7 | 15 | 18 | 31 | 38 | 45 |
| IFO 6925 | 9 | 17 | 19 | 37 | 41 | 43 |
| IFO 6930 | 8 | 10 | 18 | 29 | 36 | 45 |
| IFO 6935 | 8 | 13 | 21 | 32 | 39 | 42 |
| CM 627-2 | 7 | 14 | 20 | 33 | 44 | 44 |
| CM 627-4 | 8 | 13 | 19 | 35 | 41 | 46 |
| IFO 30604 | 6 | 11 | 20 | 35 | 39 | 40 |
| IFO 30605 | 6 | 14 | 18 | 36 | 38 | 45 |
| IFO 30606 | 6 | 14 | 18 | 30 | 36 | 44 |
| MAFF 460039 | 8 | 13 | 19 | 29 | 37 | 45 |
| KT 001 | 7 | 15 | 21 | 34 | 42 | 41 |
| IFO 6920 | 7 | 11 | 19 | 33 | 40 | 40 |
| IFO 6933 | 7 | 10 | 17 | 30 | 37 | 42 |

As shown in Table 4, the dry powder of *Tricholoma matsutake* strain FERM BP-7304, and each dry powder of 13 kinds of *Tricholoma matsutake* strains listed in Table 1 promoted the recovery of the NK cell activity. Particularly, the dry powder of *Tricholoma matsutake* strain FERM BP-7304 exhibited the most excellent activity of promoting a recovery from stress.

Further, The procedures in Example for evaluation 1(1) were repeated except that each dry powder (50 mg/kg/day) of 31 kinds of *Tricholoma matsutake* strains other than the 13 kinds of *Tricholoma matsutake* strains listed in Table 1 were used. The 31 kinds of strains were MAFF 460031, MAFF 460033, MAFF 460034, MAFF 460035, MAFF 460036, MAFF 460037, MAFF 460038, MAFF 460040, MAFF 460041, MAFF 460042, MAFF 460046, MAFF 460050, and MAFF 460096 (National Institute of Agrobiological Science; Ministry of Agriculture, Forestry and Fisheries of Japan);

CM 627-3, CM 627-5, CM 627-6, and CM 627-7(Kureha Chemical Industry Co. Ltd); and

IFO 6929, IFO 6931, IFO 6932, IFO 6934, IFO 6916, IFO 6917, IFO 6918, IFO 6919, IFO 6921, IFO 6922, IFO 6923, IFO 6924, IFO 6926, and IFO 6928 (Institute for Fermentation, Osaka). As a result, the recovery of the NK cell activity was confirmed in these *Tricholoma matsutake* strains, but no strains exhibited a more excellent activity of promoting a recovery from stress than that of *Tricholoma matsutake* strain FERM BP-7304.

(4) Evaluation for dry powder of hot water extract of *Tricholoma matsutake* fruit bodies from Nagano Prefecture and dry powder of hot water extract of various mushrooms

The procedures in Example for evaluation 1(1) were repeated except that the dry powder (prepared in Example 5) of hot water extract of *Tricholoma matsutake* fruit bodies harvested in Nagano Prefecture, or each dry powder (prepared in Comparative Examples 1 to 3, respectively) of hot water extract of *Agaricus blazei* fruit bodies, *Ganoderma lucidum (Fr.) Karst* fruit bodies, or *Lentinus edodes (Berk.) Sing.* fruit bodies were used as samples for evaluation. The dose was 250 mg/kg/day in all cases.

The results are shown in Table 5. In Table 5, the symbol "*" means $p < 0.01$ (with respect to administration of distilled water).

Table 5

| | NK cell activity (LU30) | | | | | |
|---|---|---|---|---|---|---|
| | 0day | 1day | 3days | 5days | 7days | 14days |
| Control (without stress loading) | 47 | 48 | 48 | 49 | 46 | 47 |
| Test for comparison (administration of distilled water and stress loading) | 6 | 8 | 13 | 26 | 29 | 44 |
| Example 5 | 10 | 14* | 22* | 34* | 37* | 47 |
| Comparative Examples | | | | | | |
| 1 | 5 | 7 | 15 | 27 | 30 | 37 |
| 2 | 3 | 8 | 11 | 24 | 28 | 36 |
| 3 | 4 | 9 | 10 | 27 | 29 | 40 |

INDUSTRIAL APPLICABILITY

According to the pharmaceutical composition of the present invention for promoting a recovery from stress, the recovery from stress can be promoted.

FREE TEXT IN SEQUENCE LISTING

Features of "Artificial Sequence" are described in the numeric identifier <223> in the Sequence Listing. More particularly, oligonucleotides consisting of the base sequences of SEQ ID NOS: 1 to 6 are a primer RAPD1 to a primer RAPD6, respectively.

As above, the present invention is explained with reference to particular embodiments, but modifications and improvements obvious to those skilled in the art are included in the scope of the present invention.

**Claims**

1. A pharmaceutical composition for promoting a recovery from stress, comprising *Tricholoma matsutake*, a hot water extract of *Tricholoma matsutake* or a dried product thereof, or an alkaline solution extract of *Tricholoma matsutake* or a dried product thereof, and a pharmaceutically acceptable carrier.

2. The pharmaceutical composition for promoting a recovery from stress according to claim 1, wherein the *Tricholoma matsutake* is a *Tricholoma matsutake* strain FERM BP-7304.

3. The pharmaceutical composition for promoting a recovery from stress according to claim 1 or 2, wherein the *Tricholoma matsutake* is a mycelium, a broth, or a fruit body.

4. A functional food for promoting a recovery from stress, comprising *Tricholoma matsutake*, a hot water extract of *Tricholoma matsutake* or a dried product thereof, or an alkaline solution extract of *Tricholoma matsutake* or a dried product thereof, alone or optionally with one or more food components.

5. The functional food for promoting a recovery from stress according to claim 4, wherein the *Tricholoma matsutake* is a *Tricholoma matsutake* strain FERM BP-7304.

6. The functional food for promoting a recovery from stress according to claim 4 or 5, wherein the *Tricholoma matsutake* is a mycelium, a broth, or a fruit body.

7. An oral hygienic composition for promoting a recovery from stress, comprising *Tricholoma matsutake*, a hot water extract of *Tricholoma matsutake* or a dried product thereof, or an alkaline solution extract of *Tricholoma matsutake* or a dried product thereof, and a carrier for an oral hygienic composition.

8. The oral hygienic composition for promoting a recovery from stress according to claim 7, wherein the *Tricholoma matsutake* is a *Tricholoma matsutake* strain FERM BP-7304.

9. The oral hygienic composition for promoting a recovery from stress according to claim 7 or 8, wherein the *Tricholoma matsutake* is a mycelium, a broth, or a fruit body.

10. A method for promoting a recovery from stress, comprising administering to a subject in need thereof *Tricholoma matsutake*, a hot water extract of *Tricholoma matsutake* or a dried product thereof, or an alkaline solution extract of *Tricholoma matsutake* or a dried product thereof, in an amount effective therefor.

11. The method for promoting a recovery from stress according to claim 10, wherein the *Tricholoma matsutake* is a *Tricholoma matsutake* strain FERM BP-7304.

12. The method for promoting a recovery from stress according to claim 10 or 11, wherein the *Tricholoma matsutake* is a mycelium, a broth, or a fruit body.

13. Use of *Tricholoma matsutake*, a hot water extract of *Tricholoma matsutake* or a dried product thereof, or an alkaline solution extract of *Tricholoma matsutake* or a dried product thereof, in the manufacture of a pharmaceutical composition for promoting a recovery from stress, a functional food for promoting a recovery from stress, or an oral hygienic composition for promoting a recovery from stress.

14. The use according to claim 13, wherein the *Tricholoma matsutake* is a *Tricholoma matsutake* strain FERM BP-7304.

15. The use according to claim 13 or 14, wherein the *Tricholoma matsutake* is a mycelium, a broth, or a fruit body.

16. A *Tricholoma matsutake* strain FERM BP-7304.

17. A mycelium, a broth, or a fruit body of a *Tricholoma matsutake* strain FERM BP-7304.

18. A hot water extract of a *Tricholoma matsutake* strain FERM BP-7304 or a dried product thereof, or an alkaline solution extract of a *Tricholoma matsutake* strain FERM BP-7304 or a dried product thereof.

**19.** The hot water extract or the dried product thereof, or the alkaline solution extract or the dried product thereof according to claim 18, wherein the *Tricholoma matsutake* strain FERM BP-7304 is a mycelium, a broth, or a fruit body.

FIG. 1

F I G. 2

RAPD1

RAPD4

RAPD2

RAPD5

RAPD3

RAPD6

## F I G. 3

RAPD1

RAPD4

RAPD2

RAPD5

RAPD3

RAPD6

F I G. 4

FIG. 5

FIG. 6

FIG. 7

FIG. 8

FIG. 9

## INTERNATIONAL SEARCH REPORT

| International application No. |
|---|
| PCT/JP01/08876 |

**A. CLASSIFICATION OF SUBJECT MATTER**
Int.Cl⁷   A61K35/84, 7/26, A61P3/00, A23L1/28, 1/29
C12N1/14

According to International Patent Classification (IPC) or to both national classification and IPC

**B. FIELDS SEARCHED**

Minimum documentation searched (classification system followed by classification symbols)
Int.Cl⁷   A61K35/84, 7/26, A61P3/00, A23L1/28, 1/29
C12N1/14

Documentation searched other than minimum documentation to the extent that such documents are included in the fields searched

Electronic data base consulted during the international search (name of data base and, where practicable, search terms used)
CA(STN),BIOSIS(STN),MEDLINE(STN)

**C. DOCUMENTS CONSIDERED TO BE RELEVANT**

| Category* | Citation of document, with indication, where appropriate, of the relevant passages | Relevant to claim No. |
|---|---|---|
| A | JP 2000-119126(Pola Chemical Industries Inc.), 25 April, 2000 (25.04.00)   (Family: none) | 1-9,13-19 |
| A | US 5302699 A (Director of National Food Research Institute, Ministry of Agriculture, Forestry and Fisheries), 12 April, 1994 (12.04.94), & JP 6-80699 A | 1-9,13-19 |
| A | JP 6-339351 A (Takayoshi HIRANO), 13 December, 1994 (13.12.94)   (Family: none) | 1-9,13-19 |
| A | JP 10-313876 A (Natl. Food Res. Inst.), 02 December, 1998 (02.12.98)   (Family: none) | 1-9,13-19 |
| A | JP 57-1230 B (Koji SAKAKIDA), 09 January, 1982 (09.01.82)   (Family: none) | 1-9,13-19 |

☐ Further documents are listed in the continuation of Box C.   ☐ See patent family annex.

| | |
|---|---|
| * Special categories of cited documents:<br>"A" document defining the general state of the art which is not considered to be of particular relevance<br>"E" earlier document but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| 12 December, 2001 (12.12.01) | 25 December, 2001 (25.12.01) |

| Name and mailing address of the ISA/<br>Japanese Patent Office | Authorized officer |
|---|---|
| Facsimile No. | Telephone No. |

Form PCT/ISA/210 (second sheet) (July 1992)

## INTERNATIONAL SEARCH REPORT

International application No.

PCT/JP01/08876

| Box I    Observations where certain claims were found unsearchable (Continuation of item 1 of first sheet) |
| --- |

This international search report has not been established in respect of certain claims under Article 17(2)(a) for the following reasons:

1. ☒  Claims Nos.: 10-12
because they relate to subject matter not required to be searched by this Authority, namely:

    Claims 10 to 12 pertain to methods for treatment of the human body by therapy and thus relate to a subject matter which this International Searching Authority is not required, under the provisions of Article 17(2)(a)(i) of the PCT and Rule 39(iv) of the Regulations under the PCT, to search.

2. ☐  Claims Nos.:
because they relate to parts of the international application that do not comply with the prescribed requirements to such an extent that no meaningful international search can be carried out, specifically:

3. ☐  Claims Nos.:
because they are dependent claims and are not drafted in accordance with the second and third sentences of Rule 6.4(a).

| Box II    Observations where unity of invention is lacking (Continuation of item 2 of first sheet) |
| --- |

This International Searching Authority found multiple inventions in this international application, as follows:

  The inventions as set forth in claims 1, 4, 7, 10 and 13 are method inventions based on the effect of promoting recovery from stress loading exerted by *matsutake* mushroom or optionally dried extract thereof.  On the other hand, the invention as set forth in claims 2, 3, 5, 6, 8, 9, 11, 12 and 14 to 19 are product inventions or method inventions based on a specific *matsutake* mushroom strain FERM BP-7304 *per se* or its effect as described above.  Namely, the former group consists of inventions characterized by the use of *matsutake* mushroom, while the latter group consists of inventions characterized by the specific *matsutake* mushroom strain. Such being the case, these two groups of inventions are not considered as relating to a group of inventions so linked as to form a single general inventive concept.

1. ☒  As all required additional search fees were timely paid by the applicant, this international search report covers all searchable claims.

2. ☐  As all searchable claims could be searched without effort justifying an additional fee, this Authority did not invite payment of any additional fee.

3. ☐  As only some of the required additional search fees were timely paid by the applicant, this international search report covers only those claims for which fees were paid, specifically claims Nos.:

4. ☐  No required additional search fees were timely paid by the applicant. Consequently, this international search report is restricted to the invention first mentioned in the claims; it is covered by claims Nos.:

**Remark on Protest**  ☐   The additional search fees were accompanied by the applicant's protest.
                 ☒   No protest accompanied the payment of additional search fees.

Form PCT/ISA/210 (continuation of first sheet (1)) (July 1992)

SEQUENCE LISTING

<110> Kureha Chemical Industry Co., Ltd.

<120> Pharmaceutical composition for promoting recovery from stress and novel Tricholoma matsutake strain

<130> KRH-655

<150> JP 2000-311034
<151> 2000-10-11

<150> JP 2000-311035
<151> 2000-10-11

<160> 6

<210> 1
<211> 10
<212> DNA
<213> Artificial Sequence

<220>
<223> Description of Artificial Sequence: primer RAPD1

<400> 1
tggtcaccga                                                              10

<210> 2
<211> 10
<212> DNA
<213> Artificial Sequence

<220>
<223> Description of Artificial Sequence: primer RAPD2

<400> 2
agcgccattg                                                              10

<210> 3
<211> 10
<212> DNA
<213> Artificial Sequence

<220>
<223> Description of Artificial Sequence: primer RAPD3

<400> 3
ttcgagccag                                                          10

<210> 4
<211> 10
<212> DNA
<213> Artificial Sequence

<220>
<223> Description of Artificial Sequence: primer RAPD4

<400> 4
tgcgtgcttg                                                          10

<210> 5
<211> 10
<212> DNA
<213> Artificial Sequence

<220>
<223> Description of Artificial Sequence: primer RAPD5

<400> 5
gactagcctc                                                          10

<210> 6
<211> 10
<212> DNA
<213> Artificial Sequence

<220>
<223> Description of Artificial Sequence: primer RAPD6

<400> 6
ctcaccgtcc                                                          10